# EUROPEAN PATENT APPLICATION

(11) **EP 4 847 069 A1**
(43) Date of publication of application: **02.12.2026**
(21) Application number: 26182113.6
(22) Date of filing: 29.05.2026
(51) Int. Cl.: A61M 16/00, A61N 1/36, A61B 5/087

(54) **CONTROLLING DIAPHRAGMATIC PACING, VENTILATION SYSTEM AND VENTILATION DEVICE**

(30) Priority: 29.05.2025 CN 202510718413
(71) Applicant: Shenzhen Mindray Bio-Medical Electronics Co., Ltd., Shenzhen, Guangdong 518057 (CN); Zhongda Hospital Southeast University, Nanjing, Jiangsu (CN)
(72) Inventor: LIU, Jinglei, Shenzhen, 518057 (CN); LIU, Ling, Shenzhen, 518057 (CN); YAO, Pulin, Shenzhen, 518057 (CN); YUAN, Xueyan, Shenzhen, 518057 (CN); ZHOU, Xiaoyong, Shenzhen, 518057 (CN); SUN, Qin, Shenzhen, 518057 (CN)
(74) Representative: KIPA AB

(57) **Abstract**

Disclosed are a method for controlling diaphragmatic pacing, a ventilation system and a ventilation device. While performing mechanical ventilation and diaphragmatic pacing on a patient, acquire a ventilation parameter related to the mechanical ventilation or acquire a phrenic electrical signal of the patient, then obtain a respiratory drive parameter that characterizes a degree of a respiratory effort of the patient according to the ventilation parameter or the phrenic electrical signal; and then control an intensity of the diaphragmatic pacing based on the respiratory drive parameter, so as to maintain the respiratory drive parameter within a preset range. In such a way, an automatic and accurate control of diaphragmatic pacing is realized, so as to achieve effective diaphragmatic training.

## Description

### CROSS-REFERENCE TO RELATED APPLICATIONS

This application claims priority to a Chinese patent application filed to CPO on May 29, 2025, with an application number 202510718413.1, and titled "METHOD FOR CONTROLLING DIAPHRAGMATIC PACING, VENTILATION SYSTEM AND VENTILATION DEVICE", the entire contents of which are incorporated by reference into this disclosure.

### TECHNICAL FIELD

The disclosure relates to a medical field, and more particularly to a method for controlling diaphragmatic pacing, a ventilation system and a ventilation device.

### BACKGROUND

Mechanical ventilation is one of most critical life support means for saving lives of critically ill patients, which can assist patients with respiratory failure in maintaining normal oxygenation and carbon dioxide excretion. At present, a mainstream clinical mechanical ventilation mode is positive pressure ventilation, that is, delivering ventilation support with a certain volume or pressure to patients during inhalation, and maintaining a certain positive end-expiratory pressure during exhalation to prevent alveolar collapse.

Clinical studies have shown that while maintaining gas exchange in a patient, positive pressure ventilation also causes certain damage to a respiratory system of the patient. For example, an excessive transpulmonary pressure generated by positive pressure ventilation may trigger ventilator-generated lung injury, thereby increasing a mortality of critically ill patient. Long-term positive pressure ventilation leads to disuse atrophy of diaphragm of the patient, which further prolongs a duration of ventilator support and delays ventilator weaning.

Current clinical lung protection strategies, such as low tidal volume ventilation, plateau pressure limitation and drive pressure restriction, are only effective when a patient has no spontaneous respiration or has weak spontaneous respiration. When spontaneous respiration of a patient is excessively strong, a transpulmonary pressure of the patient remains at a high level, even when conditions, such as a low tidal volume and a low drive pressure, are satisfied, which means a risk of lung injury is still high. There is currently no effective ventilation strategy for diaphragm protection.

Clinically, diaphragmatic pacing is adopted to stimulate a phrenic nerve of a patient to realize diaphragmatic contraction for diaphragmatic training, which can reduce disuse atrophy of the diaphragm to a certain extent. However, an stimulation intensity of the phrenic nerve cannot be quantitatively determined at present, and a magnitude of stimulation current or of stimulation voltage is merely set based on doctor experience. Excessively low stimulation intensity fails to effectively induce diaphragmatic contraction and cannot achieve the effect of diaphragmatic training; conversely, excessive high stimulation intensity causes overactive diaphragmatic contraction and results in diaphragm fatigue.

Therefore, the existing method for controlling diaphragmatic pacing needs to be further improved and optimized.

### SUMMARY

This disclosure mainly provides a method for controlling diaphragmatic pacing, a ventilation system and ventilation device, aiming to realize an automatic and accurate control of diaphragmatic pacing for diaphragmatic training.

In an embodiment, a method for controlling diaphragmatic pacing is provided, including:
while performing mechanical ventilation and diaphragmatic pacing on a patient, acquiring a ventilation parameter related to the mechanical ventilation or acquiring a phrenic electrical signal of the patient; wherein the phrenic electrical signal includes a diaphragmatic electrical signal or an electrical signal of a phrenic nerve;
obtaining a respiratory drive parameter that characterizes a degree of a respiratory effort of the patient according to the ventilation parameter or the phrenic electrical signal; and
controlling an intensity of the diaphragmatic pacing based on the respiratory drive parameter, so as to maintain the respiratory drive parameter within a preset range.

In the method for controlling diaphragmatic pacing provided in an embodiment, controlling an intensity of the diaphragmatic pacing based on the respiratory drive parameter, includes:
performing a closed-loop control on the intensity of the diaphragmatic pacing based on the respiratory drive parameter and the preset range.

In the method for controlling diaphragmatic pacing provided in an embodiment, the ventilation parameter includes an airway pressure and a gas flow rate, while performing the mechanical ventilation on the patient; the respiratory drive parameter includes a characteristic quantity of a respiratory muscle pressure;
obtaining a respiratory drive parameter that characterizes a degree of a respiratory effort of the patient according to the ventilation parameter, includes:
obtaining a ventilation volume according to the gas flow rate; and
obtaining the characteristic quantity of the respiratory muscle pressure, according to a preset respiratory mechanics equation, the airway pressure, the gas flow rate and the ventilation volume; wherein the respiratory mechanics equation is constructed at least based on the airway pressure, the respiratory muscle pressure, the gas flow rate, the ventilation volume and a total positive end-expiratory pressure.

In the method for controlling diaphragmatic pacing provided in an embodiment, in the respiratory mechanics equation, the respiratory muscle pressure has a preset functional relationship with time;
obtaining the characteristic quantity of the respiratory muscle pressure, according to a preset respiratory mechanics equation, the airway pressure, the gas flow rate and the ventilation volume, includes:
substituting multiple groups of the airway pressure, the gas flow rate and the ventilation volume at different time points respectively into the preset respiratory mechanics equation, so as to establish a system of equations that corresponds to the respiratory muscle pressure and the total positive end-expiratory pressure;
solving the system of equations to obtain the respiratory muscle pressure, and obtaining the characteristic quantity of the respiratory muscle pressure according to the respiratory muscle pressure.

In the method for controlling diaphragmatic pacing provided in an embodiment, the ventilation parameter includes an esophageal pressure and chest wall compliance; the respiratory drive parameter includes a characteristic quantity of a respiratory muscle pressure;
obtaining a respiratory drive parameter that characterizes a degree of a respiratory effort of the patient according to the ventilation parameter, includes:
calculating the respiratory muscle pressure according to the esophageal pressure and the chest wall compliance, and obtaining the characteristic quantity of the respiratory muscle pressure according to the respiratory muscle pressure.

In the method for controlling diaphragmatic pacing provided in an embodiment, the respiratory drive parameter includes an intensity of the phrenic electrical signal;
obtaining a respiratory drive parameter that characterizes a degree of a respiratory effort of the patient according to the phrenic electrical signal, includes:
obtaining the intensity of the phrenic electrical signal according to the phrenic electrical signal.

In the method for controlling diaphragmatic pacing provided in an embodiment, acquiring a phrenic electrical signal of the patient, includes:
measuring the diaphragmatic electrical signal of the patient via a measurement catheter that is placed inside an esophagus of the patient, when the phrenic electrical signal includes the diaphragmatic electrical signal;
measuring the diaphragmatic electrical signal of the patient via an electrode pad that is attached to an abdomen of the patient, when the phrenic electrical signal includes the diaphragmatic electrical signal;
measuring the electrical signal of the phrenic nerve of the patient via a central venous catheter with an electrode that is arranged on an outer wall of the central venous catheter, when the phrenic electrical signal includes the electrical signal of the phrenic nerve;
measuring the electrical signal of the phrenic nerve of the patient via an electrode pad that is arranged at a position of a body surface of the patient, which position corresponds to the phrenic nerve of the patient, when the phrenic electrical signal includes the electrical signal of the phrenic nerve.

In the method for controlling diaphragmatic pacing provided in an embodiment, the phrenic electrical signal include the diaphragmatic electrical signal;
the method further includes:
determining whether the diaphragm of the patient is in a fatigue state according to the respiratory drive parameter and/or the diaphragmatic electrical signal, and decreasing the intensity of the diaphragmatic pacing, if the diaphragm is in the fatigue state.

In the method for controlling diaphragmatic pacing provided in an embodiment, the phrenic electrical signal include the diaphragmatic electrical signal;
the method further includes:
determining whether the diaphragm of the patient is in a fatigue state according to the respiratory drive parameter and/or the diaphragmatic electrical signal; and suspending the closed-loop control on the intensity of the diaphragmatic pacing and decreasing the intensity of the diaphragmatic pacing, if the diaphragm is in the fatigue state;
resuming the closed-loop control on the intensity of the diaphragmatic pacing, after the diaphragm is no longer in the fatigue state.

In the method for controlling diaphragmatic pacing provided in an embodiment, performing a closed-loop control on the intensity of the diaphragmatic pacing based on the respiratory drive parameter and the preset range, includes:
decreasing the intensity of the diaphragmatic pacing, when the respiratory drive parameter exceeds an upper limit of the preset range;
increasing the intensity of the diaphragmatic pacing, when the respiratory drive parameter falls below a lower limit of the preset range.

In the method for controlling diaphragmatic pacing provided in an embodiment, increasing the intensity of the diaphragmatic pacing, when the respiratory drive parameter falls below a lower limit of the preset range, includes:
determining a target intensity that is greater than a current intensity of the diaphragmatic pacing, when the respiratory drive parameter falls below the lower limit of the preset range;
determining whether the target intensity exceeds a preset maximum intensity;
performing the diaphragmatic pacing according to the preset maximum intensity, so as to increase the intensity of the diaphragmatic pacing, if the target intensity exceeds the preset maximum intensity; or
performing the diaphragmatic pacing according to the target intensity, so as to increase the intensity of the diaphragmatic pacing, if the target intensity does not exceed the preset maximum intensity.

In an embodiment, another method for controlling diaphragmatic pacing is provided, including:
while performing mechanical ventilation and diaphragmatic pacing on a patient, acquiring a respiratory drive parameter that characterizes a degree of a respiratory effort of a patient and/or acquiring a diaphragmatic electrical signal of a patient;
determining whether the diaphragm of the patient is in a fatigue state according to the respiratory drive parameter and/or the diaphragmatic electrical signal, and decreasing an intensity of the diaphragmatic pacing, if the diaphragm is in the fatigue state.

In the method for controlling diaphragmatic pacing provided in an embodiment, decreasing the intensity of the diaphragmatic pacing, includes:
gradually decreasing the intensity of the diaphragmatic pacing.

In the method for controlling diaphragmatic pacing provided in an embodiment, the method further includes:
outputting prompt information for indicating diaphragm fatigue, when the diaphragm is in the fatigue state.

In the method for controlling diaphragmatic pacing provided in an embodiment, determining whether the diaphragm of the patient is in a fatigue state according to the respiratory drive parameter and/or the diaphragmatic electrical signal, includes:
performing a spectral analysis on the diaphragmatic electrical signal, so as to obtain a low-frequency component and a high-frequency component of the diaphragmatic electrical signal; and
determining whether a ratio of the high-frequency component to the low-frequency component is less than a preset first ratio, and determining that the diaphragm is in the fatigue state, if said ratio is less than the preset first ratio.

In the method for controlling diaphragmatic pacing provided in an embodiment, determining whether the diaphragm of the patient is in a fatigue state according to the respiratory drive parameter and/or the diaphragmatic electrical signal, includes:
calculating a ratio of the respiratory drive parameter to the intensity of the diaphragmatic electrical signal; and
determining whether the diaphragm is in the fatigue state according to an amplitude of change for said ratio.

In the method for controlling diaphragmatic pacing provided in an embodiment, determining whether the diaphragm is in the fatigue state according to an amplitude of change for said ratio, includes:
calculating the amplitude of change for said ratio by comparing said ratio with an initial ratio; or calculating the amplitude of change for said ratio by comparing said ratio with a preset reference ratio; wherein the initial ratio is calculated according to the respiratory drive parameter and the intensity of the diaphragmatic electrical signal when starting to output a stimulation signal to the patient;
determining that the diaphragm is in the fatigue state, when the amplitude of change exceeds a preset amplitude threshold.

In an embodiment, a ventilation system is provided, including a ventilation device, a diaphragmatic pacing device and a processor;
the ventilation device is configured to perform mechanical ventilation on a patient;
the diaphragmatic pacing device is configured to output a stimulation signal to the patient, so as to perform diaphragmatic pacing on the patient under a stimulation of the stimulation signal;
while the ventilation device performs the mechanical ventilation on the patient and the diaphragmatic pacing device performs the diaphragmatic pacing on the patient, the processor is configured to:
   acquire a ventilation parameter related to the mechanical ventilation or acquire a phrenic electrical signal of the patient; wherein the phrenic electrical signal includes a diaphragmatic electrical signal or an electrical signal of a phrenic nerve;
   obtain a respiratory drive parameter that characterizes a degree of a respiratory effort of the patient according to the ventilation parameter or the phrenic electrical signal; and
   control an intensity of the diaphragmatic pacing for the diaphragmatic pacing device to perform the diaphragmatic pacing based on the respiratory drive parameter, so as to maintain the respiratory drive parameter within a preset range.

In an embodiment, another ventilation system is provided, including a ventilation device, a diaphragmatic pacing device and a processor;
the ventilation device is configured to perform mechanical ventilation on a patient;
the diaphragmatic pacing device is configured to output a stimulation signal to the patient, so as to perform diaphragmatic pacing on the patient under a stimulation of the stimulation signal;
while the ventilation device performs the mechanical ventilation on the patient and the diaphragmatic pacing device performs the diaphragmatic pacing on the patient, the processor is configured to:
   acquire a respiratory drive parameter that characterizes a degree of a respiratory effort of the patient and/or acquire a diaphragmatic electrical signal of the patient;
   determine whether the diaphragm is in a fatigue state according to the respiratory drive parameter and/or the diaphragmatic electrical signal, and decrease the intensity of the diaphragmatic pacing for the diaphragmatic pacing device to perform the diaphragmatic pacing, if the diaphragm is in the fatigue state.

In an embodiment, a ventilation device is provided, including:
a sensor, which is configured to acquire a ventilation parameter related to mechanical ventilation or acquire a phrenic electrical signal of a patient; wherein the phrenic electrical signal includes a diaphragmatic electrical signal or an electrical signal of a phrenic nerve;
a breathing circuit, which is configured to deliver a gas supplied by a gas source to the patient for performing the mechanical ventilation;
a communication module, which is configured to establish a communication connection between the ventilation device and a diaphragmatic pacing device; wherein the diaphragmatic pacing device is configured to output a stimulation signal to the patient, so as to perform, diaphragmatic pacing under a stimulation of the stimulation signal; and
a processor;
while the breathing circuit performs the mechanical ventilation on the patient and the diaphragmatic pacing device performs the diaphragmatic pacing on the patient, the processor is configured to:
   acquire, via the sensor, the ventilation parameter related to mechanical ventilation or the phrenic electrical signal of the patient;
   obtain a respiratory drive parameter that characterizes a degree of a respiratory effort of the patient according to the ventilation parameter or the phrenic electrical signal; and
   control an intensity of the diaphragmatic pacing for the diaphragmatic pacing device to perform the diaphragmatic pacing based on the respiratory drive parameter, so as to maintain the respiratory drive parameter within a preset range.

In an embodiment, a ventilation device is provided, including:
a sensor, which is configured to acquire a ventilation parameter related to mechanical ventilation of a patient or acquire a diaphragmatic electrical signal of the patient;
a breathing circuit, which is configured to deliver a gas supplied by a gas source to the patient for performing the mechanical ventilation;
a communication module, which is configured to establish a communication connection between the ventilation device and a diaphragmatic pacing device; wherein the diaphragmatic pacing device is configured to output a stimulation signal to the patient, so as to perform diaphragmatic pacing on the patient under a stimulation of the stimulation signal; and
a processor;
while the breathing circuit performs the mechanical ventilation on the patient and the diaphragmatic pacing device performs the diaphragmatic pacing on the patient, the processor is configured to:
   acquire, via the sensor, the ventilation parameter related to mechanical ventilation or the diaphragmatic electrical signal of the patient;
   obtain a respiratory drive parameter that characterizes a degree of a respiratory effort of the patient according to the ventilation parameter or the diaphragmatic electrical signal;
   determine whether the diaphragm is in a fatigue state according to the respiratory drive parameter and the diaphragmatic electrical signal, and decreasing an intensity of the diaphragmatic pacing for the diaphragmatic pacing device to perform the diaphragmatic pacing, if the diaphragm is in the fatigue state; or determine whether the diaphragm is in a fatigue state according to the diaphragmatic electrical signal, and decreasing an intensity of the diaphragmatic pacing for the diaphragmatic pacing device to perform the diaphragmatic pacing, if the diaphragm is in the fatigue state.

In an embodiment, a computer-readable storage medium is provided, wherein a computer program is stored on the computer-readable storage medium, and the computer program is capable of being executed by a processor, so as to implement the method as described above.

In an embodiment, a computer-readable storage medium is provided, wherein a computer program is stored on the computer-readable storage medium, and the computer program is capable of being executed by a processor, so as to implement a method for controlling diaphragmatic pacing, including:
while performing mechanical ventilation and diaphragmatic pacing on a patient, acquiring a ventilation parameter related to the mechanical ventilation or acquiring a phrenic electrical signal of the patient; wherein the phrenic electrical signal includes a diaphragmatic electrical signal or an electrical signal of a phrenic nerve;
obtaining a respiratory drive parameter that characterizes a degree of a respiratory effort of the patient according to the ventilation parameter or the phrenic electrical signal; and
controlling an intensity of the diaphragmatic pacing based on the respiratory drive parameter, so as to maintain the respiratory drive parameter within a preset range.

In the computer-readable storage medium provided in an embodiment, controlling an intensity of the diaphragmatic pacing based on the respiratory drive parameter, includes:
performing a closed-loop control on the intensity of the diaphragmatic pacing based on the respiratory drive parameter and the preset range.

In the computer-readable storage medium provided in an embodiment, the ventilation parameter includes an airway pressure and a gas flow rate, while performing the mechanical ventilation on the patient; the respiratory drive parameter includes a characteristic quantity of a respiratory muscle pressure;
obtaining a respiratory drive parameter that characterizes a degree of a respiratory effort of the patient according to the ventilation parameter, includes:
obtaining a ventilation volume according to the gas flow rate; and
obtaining the characteristic quantity of the respiratory muscle pressure, according to a preset respiratory mechanics equation, the airway pressure, the gas flow rate and the ventilation volume; wherein the respiratory mechanics equation is constructed at least based on the airway pressure, the respiratory muscle pressure, the gas flow rate, the ventilation volume and a total positive end-expiratory pressure.

In the computer-readable storage medium provided in an embodiment, in the respiratory mechanics equation, the respiratory muscle pressure has a preset functional relationship with time;
obtaining the characteristic quantity of the respiratory muscle pressure, according to a preset respiratory mechanics equation, the airway pressure, the gas flow rate and the ventilation volume, includes:
substituting multiple groups of the airway pressure, the gas flow rate and the ventilation volume at different time points respectively into the preset respiratory mechanics equation, so as to establish a system of equations that corresponds to the respiratory muscle pressure and the total positive end-expiratory pressure;
solving the system of equations to obtain the respiratory muscle pressure, and obtaining the characteristic quantity of the respiratory muscle pressure according to the respiratory muscle pressure.

In the computer-readable storage medium provided in an embodiment, the ventilation parameter includes an esophageal pressure and chest wall compliance; the respiratory drive parameter includes a characteristic quantity of a respiratory muscle pressure;
obtaining a respiratory drive parameter that characterizes a degree of a respiratory effort of the patient according to the ventilation parameter, includes:
calculating the respiratory muscle pressure according to the esophageal pressure and the chest wall compliance, and obtaining the characteristic quantity of the respiratory muscle pressure according to the respiratory muscle pressure.

In the computer-readable storage medium provided in an embodiment, the respiratory drive parameter includes an intensity of the phrenic electrical signal;
obtaining a respiratory drive parameter that characterizes a degree of a respiratory effort of the patient according to the phrenic electrical signal, includes:
obtaining the intensity of the phrenic electrical signal according to the phrenic electrical signal.

In the computer-readable storage medium provided in an embodiment, acquiring a phrenic electrical signal of the patient, includes:
measuring the diaphragmatic electrical signal of the patient via a measurement catheter that is placed inside an esophagus of the patient, when the phrenic electrical signal includes the diaphragmatic electrical signal;
measuring the diaphragmatic electrical signal of the patient via an electrode pad that is attached to an abdomen of the patient, when the phrenic electrical signal includes the diaphragmatic electrical signal;
measuring the electrical signal of the phrenic nerve of the patient via a central venous catheter with an electrode that is arranged on an outer wall of the central venous catheter, when the phrenic electrical signal includes the electrical signal of the phrenic nerve;
measuring the electrical signal of the phrenic nerve of the patient via an electrode pad that is arranged at a position of a body surface of the patient, which position corresponds to the phrenic nerve of the patient, when the phrenic electrical signal includes the electrical signal of the phrenic nerve.

In the computer-readable storage medium provided in an embodiment, the phrenic electrical signal include the diaphragmatic electrical signal;
the method further includes:
determining whether the diaphragm of the patient is in a fatigue state according to the respiratory drive parameter and/or the diaphragmatic electrical signal, and decreasing the intensity of the diaphragmatic pacing, if the diaphragm is in the fatigue state.

In the computer-readable storage medium provided in an embodiment, the phrenic electrical signal include the diaphragmatic electrical signal;
the method further includes:
determining whether the diaphragm of the patient is in a fatigue state according to the respiratory drive parameter and/or the diaphragmatic electrical signal; and suspending the closed-loop control on the intensity of the diaphragmatic pacing and decreasing the intensity of the diaphragmatic pacing, if the diaphragm is in the fatigue state;
resuming the closed-loop control on the intensity of the diaphragmatic pacing, after the diaphragm is no longer in the fatigue state.

In the computer-readable storage medium provided in an embodiment, performing a closed-loop control on the intensity of the diaphragmatic pacing based on the respiratory drive parameter and the preset range, includes:
decreasing the intensity of the diaphragmatic pacing, when the respiratory drive parameter exceeds an upper limit of the preset range;
increasing the intensity of the diaphragmatic pacing, when the respiratory drive parameter falls below a lower limit of the preset range.

In the computer-readable storage medium provided in an embodiment, increasing the intensity of the diaphragmatic pacing, when the respiratory drive parameter falls below a lower limit of the preset range, includes:
determining a target intensity that is greater than a current intensity of the diaphragmatic pacing, when the respiratory drive parameter falls below the lower limit of the preset range;
determining whether the target intensity exceeds a preset maximum intensity;
performing the diaphragmatic pacing according to the preset maximum intensity, so as to increase the intensity of the diaphragmatic pacing, if the target intensity exceeds the preset maximum intensity; or
performing the diaphragmatic pacing according to the target intensity, so as to increase the intensity of the diaphragmatic pacing, if the target intensity does not exceed the preset maximum intensity.

In an embodiment, a computer-readable storage medium is provided, wherein a computer program is stored on the computer-readable storage medium, and the computer program is capable of being executed by a processor, so as to implement a method for controlling diaphragmatic pacing, including:
while performing mechanical ventilation and diaphragmatic pacing on a patient, acquiring a respiratory drive parameter that characterizes a degree of a respiratory effort of a patient and/or acquiring a diaphragmatic electrical signal of a patient;
determining whether the diaphragm of the patient is in a fatigue state according to the respiratory drive parameter and/or the diaphragmatic electrical signal, and decreasing an intensity of the diaphragmatic pacing, if the diaphragm is in the fatigue state.

In the computer-readable storage medium provided in an embodiment, decreasing the intensity of the diaphragmatic pacing, includes:
gradually decreasing the intensity of the diaphragmatic pacing.

In the computer-readable storage medium provided in an embodiment, the method further includes:
outputting prompt information for indicating diaphragm fatigue, when the diaphragm is in the fatigue state.

In the computer-readable storage medium provided in an embodiment, determining whether the diaphragm of the patient is in a fatigue state according to the respiratory drive parameter and/or the diaphragmatic electrical signal, includes:
performing a spectral analysis on the diaphragmatic electrical signal, so as to obtain a low-frequency component and a high-frequency component of the diaphragmatic electrical signal; and
determining whether a ratio of the high-frequency component to the low-frequency component is less than a preset first ratio, and determining that the diaphragm is in the fatigue state, if said ratio is less than the preset first ratio.

In the computer-readable storage medium provided in an embodiment, determining whether the diaphragm of the patient is in a fatigue state according to the respiratory drive parameter and/or the diaphragmatic electrical signal, includes:
calculating a ratio of the respiratory drive parameter to the intensity of the diaphragmatic electrical signal; and
determining whether the diaphragm is in the fatigue state according to an amplitude of change for said ratio.

In the computer-readable storage medium provided in an embodiment, determining whether the diaphragm is in the fatigue state according to an amplitude of change for said ratio, includes:
calculating the amplitude of change for said ratio by comparing said ratio with an initial ratio; or calculating the amplitude of change for said ratio by comparing said ratio with a preset reference ratio; wherein the initial ratio is calculated according to the respiratory drive parameter and the intensity of the diaphragmatic electrical signal when starting to output a stimulation signal to the patient;
determining that the diaphragm is in the fatigue state, when the amplitude of change exceeds a preset amplitude threshold.

According to the method for controlling diaphragmatic pacing, the ventilation system and the ventilation device of the above embodiments, while performing mechanical ventilation and diaphragmatic pacing on a patient, acquire a ventilation parameter related to the mechanical ventilation or acquire a phrenic electrical signal of the patient, then obtain a respiratory drive parameter that characterizes a degree of a respiratory effort of the patient according to the ventilation parameter or the phrenic electrical signal; and then control an intensity of the diaphragmatic pacing based on the respiratory drive parameter, so as to maintain the respiratory drive parameter within a preset range. In such a way, an automatic and accurate control of diaphragmatic pacing is realized, so as to achieve effective diaphragmatic training.

### BRIEF DESCRIPTION OF THE DRAWINGS

FIG. 1 is a flowchart for an embodiment of a method for controlling diaphragmatic pacing provided by this disclosure.
FIG. 2 is a flowchart for another embodiment of a method for controlling diaphragmatic pacing provided by this disclosure.
FIG. 3 is a structural block diagram for an embodiment of a ventilation system provided by this disclosure.
FIG. 4 is a structural block diagram for another embodiment of a ventilation system provided by this disclosure.
FIG. 5 is a structural block diagram for a still another embodiment of a ventilation system provided by this disclosure;
FIG. 6 is a flowchart for an embodiment of Step 2 in FIG. 1.
FIG. 7 is a waveform curve diagram for an airway pressure, a respiratory muscle pressure, a gas flow rate and a ventilation volume.
FIG. 8 is a schematic diagram for modeling a respiratory system.
FIG. 9 is a waveform curve diagram for an airway pressure, an esophageal pressure and a gas flow rate.
FIG. 10 is a structural block diagram for an embodiment of a ventilation device.

### DESCRIPTION OF THE PREFERRED EMBODIMENTS

This disclosure will be described in further detail below in conjunction with specific embodiments and accompanying drawings. In different embodiments, similar components adopt associated and similar component numbers. In the following embodiments, numerous detailed descriptions are provided to facilitate a better understanding of this disclosure. However, those skilled in the art can readily recognize that some features may be omitted under different circumstances, or be replaced by other components, materials and methods. In some cases, certain operations related to this disclosure are not shown or described in the description, so as to prevent the core part of this disclosure from being obscured by excessive descriptions. For those skilled in the art, detailed descriptions of these related operations are not essential, and they can fully understand the relevant operations based on the content of the description and general common technical knowledge in the field.

In addition, the features, operations or characteristics described in the description may be combined in any appropriate manner to form various embodiments. Meanwhile, each step or action recorded in the method description may be reordered or adjusted in a manner obvious to those skilled in the art. Therefore, sequences presented in the description and accompanying drawings are merely for clearly describing a certain embodiment, and do not represent a mandatory order unless otherwise specified.

The serial numbers such as "first" and "second" assigned to components in this text are only used to distinguish described objects, without any sequential or technical implication. The terms "connected" and "coupled" as mentioned in this disclosure include direct and indirect connection (coupling) unless otherwise specified.

The diaphragm is the most important inspiratory muscle. This disclosure proposes a ventilation method combining a ventilator and diaphragmatic pacing based on a respiratory effort. This regulation strategy ensures that a respiratory effort of the patient during diaphragmatic pacing is maintained within a clinically reasonable range, so as to reduce lung injury or diaphragm fatigue caused by excessive spontaneous respiration, as well as disuse atrophy of the diaphragm caused by persistently insufficient spontaneous respiration. Detailed descriptions will be given below in conjunction with several embodiments.

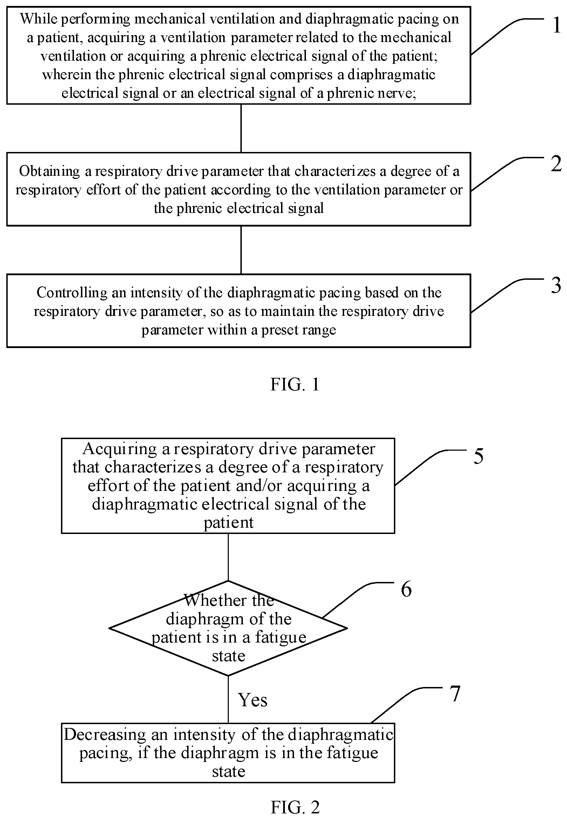

As shown in FIG. 1, the method for controlling diaphragmatic pacing according to an embodiment of this disclosure may include following steps.

Step 1: while performing mechanical ventilation and diaphragmatic pacing on a patient, acquire a ventilation parameter related to the mechanical ventilation or acquire a phrenic electrical signal of the patient. Wherein the phrenic electrical signal includes a diaphragmatic electrical signal or an electrical signal of a phrenic nerve.

Step 2: obtain a respiratory drive parameter that characterizes a degree of a respiratory effort of the patient according to the ventilation parameter or the phrenic electrical signal. That is, quantify a degree of a respiratory effort of the patient through the ventilation parameter or the phrenic electrical signal.

Step 3: control an intensity of the diaphragmatic pacing based on the respiratory drive parameter, so as to maintain the respiratory drive parameter within a preset range. Equivalently, the diaphragmatic pacing is controlled or regulated according to the degree of the respiratory effort of the patient, so as to maintain the respiratory effort of the patient within a certain range.

For a patient who receives mechanical ventilation, diaphragmatic pacing functions to contract the diaphragm. In general, an intensity of an electrical or magnetic stimulation applied to a phrenic nerve or a diaphragm is positively correlated with an intensity of diaphragmatic contraction. Excessively low stimulation intensity cannot trigger the diaphragmatic contraction, or may lead to inadequate diaphragmatic contraction, failing to achieve a purpose of diaphragmatic training. Excessively high stimulation intensity may cause excessive diaphragmatic contraction and further result in diaphragm fatigue. In the prior art, when performing diaphragmatic pacing, the intensity of the diaphragmatic pacing is usually set by clinicians according to their experience. Long-term diaphragmatic pacing is required for effective diaphragmatic training. Conventional solutions perform diaphragmatic pacing at a fixed intensity set by a doctor, which suffers from poor accuracy. The stimulation intensity may be insufficient to induce effective diaphragmatic contraction, or may be excessively high to trigger diaphragm fatigue. Frequent manual adjustment for the intensity of the diaphragmatic pacing consumes substantial time of both medical staff and patient. Moreover, differences exist among individual patients, as well as among different physiological conditions of a same patient, such that the clinicians need to reset the stimulation intensity each time, which results in a heavy workload. In contrast, this disclosure adopts a real-time measurement of a respiratory effort generated by diaphragmatic pacing to realize a feedback control (e.g., a closed-loop feedback) on the stimulation intensity, so as to maintain an intensity of the diaphragmatic contraction stably within a clinically acceptable normal range. Maintaining an intensity of diaphragmatic pacing to a reasonable range rather than a fixed value, adapts to different patients and different physical conditions of a same patient, does not dependent on clinical experience and manual adjustment, thus not only achieving a favorable training effect for the diaphragm, but also greatly decreasing the workload of medical staff.

Another embodiment of this disclosure further provides another method for controlling diaphragmatic pacing. As shown in FIG. 2, the method may include following steps.

Step 5: while performing mechanical ventilation and diaphragmatic pacing on a patient, acquire a respiratory drive parameter that characterizes a degree of a respiratory effort of the patient and/or acquire a diaphragmatic electrical signal of the patient. The acquisition of the diaphragmatic electrical signal in this step may be the same as that in Step 1. In this step, while performing mechanical ventilation and diaphragmatic pacing on a patient, acquire a ventilation parameter related to the mechanical ventilation, acquire a phrenic electrical signal of the patient, and then obtain the respiratory drive parameter that characterizes the degree of the respiratory effort of the patient according to the ventilation parameter or the phrenic electrical signal. That is, acquiring the respiratory drive parameter may include Step 1 and Step 2.

Step 6: determine whether a diaphragm of the patient is in a fatigue state according to the respiratory drive parameter and/or the diaphragmatic electrical signal.

Step 7: if the diaphragm is in a fatigue state, decrease an intensity of the diaphragmatic pacing, so as to restore the diaphragm of the patient to a normal state.

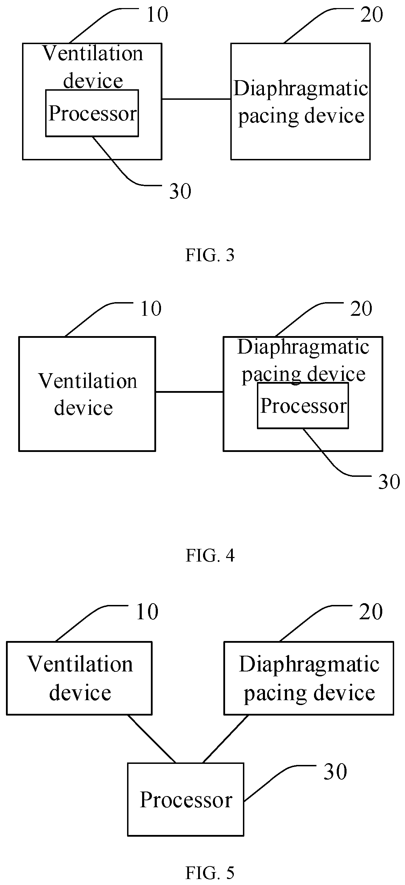

The above control method may be implemented by a user manually operating the ventilation device 10 and the diaphragmatic pacing device 20, or may be performed automatically. This embodiment is described taking the automatic implementation as an example. In this embodiment, a ventilation system controls the diaphragmatic pacing. For example, as shown in FIG. 3 to FIG. 5, the ventilation system provided by this disclosure may include a ventilation device 10, a diaphragmatic pacing device 20 and a processor 30.

The ventilation device 10 is configured to perform mechanical ventilation on a patient, so as to provide a respiratory support. The ventilation device may be any device capable of providing respiratory support for the patient, such as a ventilator, an anesthesia machine, an oxygen therapy instrument or the likes. This embodiment is described taking a ventilator as an example.

The diaphragmatic pacing device 20 is configured to perform diaphragmatic pacing on the patient, for example, outputting a stimulation signal to the patient to generate diaphragmatic contraction under a stimulation of such signal. The stimulation signal may be an electrical stimulation signal, a magnetic stimulation signal and the likes. In general, the intensity of the stimulation signal is positively correlated with the intensity of the diaphragmatic contraction. The stimulation signal may be applied to a phrenic nerve of the patient or a diaphragm of the patient, so as to achieve diaphragmatic contraction. For instance, an electrode of the diaphragmatic pacing device 20 may be attached to the phrenic nerve at the neck of the patient or an invasive electrode may be implanted on the phrenic nerve. The diaphragmatic pacing device 20 outputs the stimulation signal through the electrode to stimulate the phrenic nerve, thus triggering diaphragmatic contraction. The technical mean of stimulating the diaphragm for diaphragmatic pacing through the diaphragmatic pacing device 20 belongs to conventional art, and will not be elaborated herein.

The processor 30 is configured to control the ventilation device 10 and the diaphragmatic pacing device 20, so as to control the diaphragmatic pacing during a long-term mechanical ventilation of the patient. The processor 30 may be a processor of the ventilation device 10 as shown in FIG. 3, which means the ventilation device 10 takes the lead in controlling diaphragmatic pacing. Alternatively, the processor 30 may be the processor of the diaphragmatic pacing device 20 as shown in FIG. 4, which means the diaphragmatic pacing device 20 takes the lead in controlling diaphragmatic pacing. In addition, the processor 30 may be an independent device that is independent of the ventilation device 10 and the diaphragmatic pacing device 20 as shown in FIG. 5, that is, an independent control device equipped with the processor 30 is adopted to control both the ventilation device 10 and the diaphragmatic pacing device 20, so as to control the diaphragmatic pacing.

The processor 30 controls the diaphragmatic pacing of the patient through the ventilation device 10 and the diaphragmatic pacing device 20. Specifically, in Step 1, the ventilation device 10 performs mechanical ventilation on the patient, and the diaphragmatic pacing device 20 starts diaphragmatic pacing after receiving an instruction for diaphragmatic pacing. The instruction for diaphragmatic pacing may be issued by a clinician to manually activate the diaphragmatic pacing device 20, or automatically generated by the processor 30 after the mechanical ventilation lasts for a preset duration. When a patient uses the ventilation device 10 for a long term, the diaphragm of the patient becomes weak. When a condition of the patient improves, clinicians may activate the diaphragmatic pacing device 20 to stimulate the diaphragm for diaphragmatic training. Alternatively, the diaphragmatic pacing device 20 can be automatically started after the patient uses the ventilation device 10 for a long term, so as to implement the diaphragmatic pacing on the patient for diaphragmatic training. The ventilation device 10 is in communication connection with the diaphragmatic pacing device 20, such that the diaphragmatic pacing device 20 can acquire a respiratory cycle of the patient from the ventilation device 10 and identify inspiratory and expiratory phases (spontaneous respiration or controlled respiration) of the patient, so as to enable the diaphragmatic pacing to be synchronized with a respiratory rhythm of the patient. For example, a stimulation signal is outputted to contract the diaphragm during the inspiratory phase of the patient, in which the ventilation device 10 delivers a gas to the patient, while the stimulation signal is stopped during the expiratory phase. Certainly, in some embodiments, diaphragmatic pacing may operate asynchronously with the respiratory rhythm of the patient. While the ventilation device 10 performs mechanical ventilation on the patient, and the diaphragmatic pacing device 20 performs diaphragmatic pacing on the patient, the processor 30 acquires a ventilation parameter related to the mechanical ventilation or a phrenic electrical signal of the patient. The processor 30 may detect the ventilation parameter related to the mechanical ventilation via a sensor of the ventilation device 10, or obtain the ventilation parameter from an external device (such as a monitor) through a communication module of the ventilation device 10 or of the diaphragmatic pacing device 20. Similarly, the processor 30 may acquire a phrenic electrical signal through a sensor of the ventilation device 10 or the diaphragmatic pacing device 20, or acquire the phrenic electrical signal from an external monitoring device (e.g., a patient monitor) through a communication module of the ventilation device 10 or of the diaphragmatic pacing device 20. The phrenic electrical signal may include a diaphragmatic electrical signal, which is an electrical activity of a diaphragmatic muscle, and capable of reflecting an intensity of respiratory drive. The ventilation device 10, the diaphragmatic pacing device 20 or the monitoring device may include a measurement catheter (such as an esophageal measurement catheter), and the ventilation device 10, the diaphragmatic pacing device 20 or the monitoring device may acquire the diaphragmatic electrical signal through the measurement catheter that is placed inside an esophagus of the patient. The ventilation device 10, the diaphragmatic pacing device 20 or the monitoring device may include an electrode pad, and the ventilation device 10, the diaphragmatic pacing device 20 or the monitoring device may measure the diaphragmatic electrical signal through the electrode pad that is attached to an abdomen of the patient. The phrenic electrical signal may further include an electrical signal of a phrenic nerve, which is an electrical activity of the phrenic nerve, and capable of reflecting an intensity of respiratory drive. The ventilation device 10, the diaphragmatic pacing device 20 or the monitoring device may include a central venous catheter with an electrode on an outer wall of the central venous catheter, and the ventilation device 10, the diaphragmatic pacing device 20 or the monitoring device can measure the electrical signal of the phrenic nerve via the central venous catheter. The ventilation device 10, the diaphragmatic pacing device 20 or the monitoring device may include an electrode pad, and the ventilation device 10, the diaphragmatic pacing device 20 or the monitoring device may measure the electrical signal of the phrenic nerve of the patient through the electrode pad that is attached to a body surface of the patient at a position of the body surface, which position corresponds to the phrenic nerve. The detection of the phrenic electrical signal belongs to conventional technical means in the field, and will not be repeated herein.

In Step 2, the processor 30 obtains a respiratory drive parameter that represents a degree of a respiratory effort of the patient according to the ventilation parameter or the phrenic electrical signal. Multiple specific implementation modes are available, and three typical modes are described below.

In the first implementation mode, the ventilation parameter includes an airway pressure and a gas flow rate, while performing the mechanical ventilation on the patient; the corresponding respiratory drive parameter includes a characteristic quantity of a respiratory muscle pressure. As a quantified value, the characteristic quantity of the respiratory muscle pressure can effectively reflect a degree of a respiratory effort of the patient, as well as a strength of diaphragmatic contraction, such that said characteristic quantity is capable of feeding the diaphragmatic pacing back, performing a quantitative closed-loop control on the diaphragmatic pacing, thus avoiding poor training effect resulted by insufficient diaphragmatic contraction, as well as diaphragm fatigue resulted by excessive diaphragmatic contraction.

The sensor of the ventilation device 10 may include a pressure sensor and a flow rate sensor. The pressure sensor is arranged at a position that is connected with an airway of the patient, for example, in a breathing circuit of the ventilation device and the likes. When the ventilation device 10 performs the mechanical ventilation (provides a respiratory support) on the patient, the pressure sensor collects the airway pressure of the patient. The flow rate sensor is also arranged at a position that is connected with the airway of the patient, such as in a breathing circuit of the ventilation device and the likes. When the ventilation device 10 performs the mechanical ventilation on the patient, the flow rate sensor collects a gas flow rate of the mechanical ventilation, that is collecting the gas flow rate. The ventilation device 10 usually displays the collected airway pressure and gas flow rate on its interface, and triggers an alarm when such parameters respectively exceed threshold values. Notably, this disclosure can calculate the respiratory drive parameter by utilizing built-in (existing) airway pressure and gas flow rate of a conventional ventilator without additional parameter collection, which is convenient and efficient.

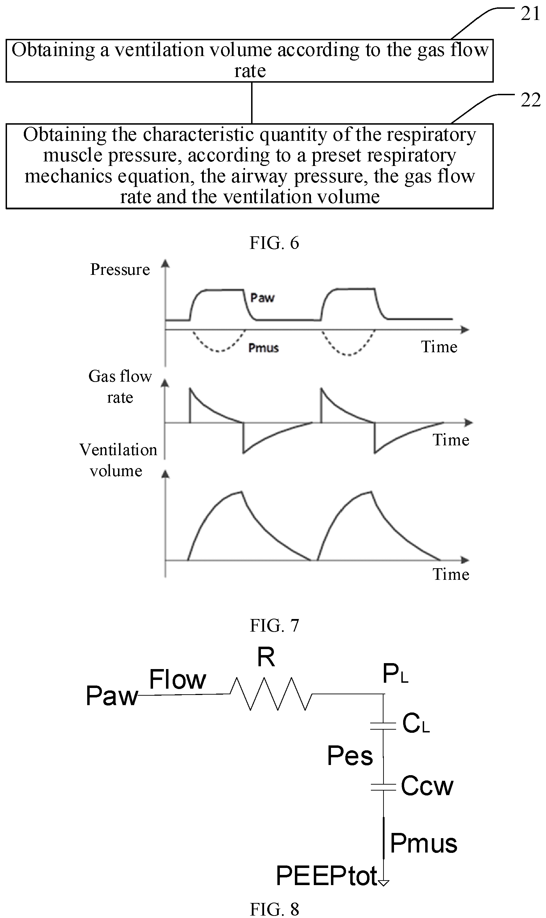

In the first implementation mode, Step 2 includes Step 21 and Step 22 as shown in FIG. 6.

Step 21: the processor 30 acquires a ventilation volume *Volume* according to a gas flow rate *Flow*. For example, the processor 30 integrates the gas flow rate *Flow* with respect to time, so as to obtain the ventilation volume *Volume*, i.e., a gas volume that is delivered by the ventilation device 10. The processor 30 may obtain a real-time ventilation volume *Volume* according to a real-time gas flow rate *Flow*.

Step 22: the processor 30 obtains the respiratory muscle pressure according to a preset respiratory mechanics equation, as well as the airway pressure, the gas flow rate and the ventilation volume, and further obtains the characteristic quantity of the respiratory muscle pressure based on the respiratory muscle pressure. The respiratory mechanics equation is constructed at least based on the airway pressure *Paw*, the respiratory muscle pressure *Pmus*, the gas flow rate *Flow*, the ventilation volume *Volume* and a total positive end-expiratory pressure *PEEPtot*.

As described above, both the airway pressure and the gas flow rate are collected and calculated during a conventional ventilation. Therefore, the detection of the characteristic quantity of the respiratory muscle pressure does not require expiratory hold operation, will not interrupt routine ventilation, and is applicable to both invasive and non-invasive ventilation modes.

The processor 30 may display the characteristic quantity of the respiratory muscle pressure on a display of the ventilation device 10 or on a display of the diaphragmatic pacing device 20 for clinician observation. Since the airway pressure and the gas flow rate can be acquired in real time by corresponding sensors, the processor 30 can calculate the ventilation volume in real-time and further obtain real-time characteristic quantity of the respiratory muscle pressure. Accordingly, the display of the characteristic quantity of the respiratory muscle pressure is in real-time, and such real-time display enables clinicians to intuitively grasp an intuitive feeling of respiratory effort of the patient.

The respiratory system can be simplified as a first-order RC system, wherein *R* refers to respiratory system resistance (such as viscous resistance of respiratory system), and *C* refers to respiratory system compliance (which may also be represented by an elastic resistance of respiratory system *E*). During mechanical ventilation, a respiratory effort mainly derives from two aspects: a drive force of the ventilator, i.e., airway pressure *Paw*, and a contraction force generated by a respiratory muscle of the respiratory system (that is, the respiratory muscle pressure) *Pmus*. Based on the first-order model, the respiratory mechanics equation is obtained as follows: $Paw - Pmus = Flow * R + Volume * E + PEEPtot .$

Wherein:
*Paw* refers to the airway pressure, which can be directly measured by an airway pressure sensor during mechanical ventilation;
*Flow* refers to the gas flow rate, which can be measured by a flow rate sensor;
*Volume* refers to the ventilation volume, which can be obtained by integrating the gas flow rate;
*PEEPtot* refers to the total positive end-expiratory pressure (that is, total *PEEP*), is equal to a sum of extrinsic positive end-expiratory pressure *PEEPe* (that is, extrinsic *PEEP*, i.e., PEEP monitored via the airway pressure) and intrinsic positive end-expiratory pressure *PEEPi* (that is, intrinsic *PEEP*);
*Pmus* refers to the respiratory muscle pressure;
*R* refers to the viscous resistance of respiratory system of the patient, and is generally a constant;
*E* refers to the elastic resistance of respiratory system of the patient, and is generally a constant.

Curve graphs of each variable in the respiratory mechanics equation are shown in FIG. 7. The aforesaid Step 22 may be implemented by solving a system of respiratory mechanics equations. Specifically, in the respiratory mechanics equation, the total positive end-expiratory pressure *PEEPtot,* the respiratory muscle pressure *Pmus,* the viscous resistance of respiratory system *R* and the elastic resistance of respiratory system *E* are unknown quantities, and the remaining parameters are known quantities. The respiratory muscle pressure *Pmus* varies with time, during an inspiration phase of the patient. By measuring a series of values for the airway pressure *Paw,* the gas flow rate *Flow* and the ventilation volume *Volume*, multiple equations can be established as follows: $Paw \left(1\right) - Pmus \left(1\right) = Flow \left(1\right) * R + Volume \left(1\right) * E + PEEPtot ,$ $Paw \left(2\right) - Pmus \left(2\right) = Flow \left(2\right) * R + Volume \left(2\right) * E + PEEPtot ,$ *......* $Paw \left(n\right) - Pmus \left(n\right) = Flow \left(n\right) * R + Volume \left(n\right) * E + PEEPtot .$

In other words, the processor 30 substitutes multiple groups of the airway pressure *Paw*, the gas flow rate *Flow* and the ventilation volume *Volume* at different time points into the preset respiratory mechanics equation as known quantities, so as to establish a system of equations with the respiratory muscle pressure *Pmus* and the total positive end-expiratory pressure *PEEPtot* as unknown quantities.

Taking *n* groups of data for the airway pressure *Paw*, the gas flow rate *Flow* and the ventilation volume *Volume* as an example, *n* equations can be obtained, while there are *n+3* unknown quantities. To make the system of equations solvable, the respiratory muscle pressure *Pmus* may be simplified. For example, in accordance with a physiological characteristic of spontaneous respiration, the respiratory muscle pressure *Pmus* is simplified into a known function (curve). That is to say, in the respiratory mechanics equation, the respiratory muscle pressure *Pmus* has a preset functional relationship with time *t*. Although the respiratory muscle pressure *Pmus* is an unknown quantity, however the respiratory muscle pressure *Pmus* exhibits regularity (having a preset functional relationship with time). The preset functional relationship may include one or a combination of: an exponential function, a trigonometric function (e.g., *sin(b₀+b₁t)*), a piecewise function and a polynomial function (e.g., parabola *a₁t+a₂t²*). Simulating the respiratory muscle pressure *Pmus* via a sine function, a cosine function, a quadratic polynomial function or the likes can reduce the number of unknown quantities to five. This embodiment takes simplifying the respiratory muscle pressure *Pmus* with a parabola (a quadratic polynomial function) as an example, and the above system of equations is simplified as follows: $Y = {\left[Paw\left(1\right),Paw\left(2\right), …, Paw\left(n\right)\right]}^{T}$ $F = {\left[Flow\left(1\right),Flow\left(2\right), …, Flow\left(n\right)\right]}^{T}$ $V = {\left[Volume\left(1\right),Volume\left(2\right), …, Volume\left(n\right)\right]}^{T}$

Let: $X = \left[F, V, 1, t, {t}^{2}\right] , A = {\left[R, E, PEEPtot, {a}_{1}, {a}_{2}\right]}^{T} .$

The above system of equations can be expressed by the matrix formula: *Y=X*A*. Solving the system of equations through matrix calculation yields the respiratory muscle pressure *Pmus*. Specifically, a number of data sampling points (groups of data for the airway pressure *Paw*, the gas flow rate *Flow* and the ventilation volume *Volume*) collected during an inspiration process is generally far more than five, accordingly the system of equations is an overdetermined system. The value of *n* satisfies a solving requirement of the *n+3* overdetermined equations. If the viscous resistance of respiratory system *R* and the elastic resistance of respiratory system *E* are known quantities, the value of *n* satisfies a solving requirement of *n+1* overdetermined equations. An optimal solution of the overdetermined system can be obtained through the following steps:
Given *Y=X*A*, then *X^{T} *Y=X^{T} *X*A*; wherein *X^{T} *X* is a 5×5 matrix that is capable of performing an inverse operation;
therefore: *A=(X^{T} *X)⁻¹ X^{T} *Y*.

Then, the respiratory muscle pressure *Pmus* can be obtained after matrix solving.

Further, one or more constraint conditions may be added, while solving the system of equations, so as to improve an accuracy and effectiveness of a calculation result. For instance, one constraint condition is that when calculating the respiratory muscle pressure *Pmus*, the processor 30 sets the respiratory muscle pressure *Pmus* at a start time point and an end time point of an inspiration phase to 0 (refer to the *Pmus* waveform in FIG. 7), and restricts the respiratory muscle pressure *Pmus* to be greater than or equal to zero. Another constraint condition is defining the viscous resistance *R* of respiratory system and the elastic resistance *E* of respiratory system within a preset reasonable range. A third constraint condition is setting the intrinsic positive end-expiratory pressure *PEEPi* to be greater than or equal to 0. A fourth constraint condition is setting the respiratory muscle pressure *Pmus* to 0 for a patient without spontaneous respiration. One or more of the above four constraint conditions may be selected for solving the system of equations, so as to calculate the respiratory muscle pressure *Pmus* more accurately and reliably.

The processor 30 acquires the characteristic quantity of the respiratory muscle pressure based on the calculated respiratory muscle pressure *Pmus*. As shown in FIG. 7, the respiratory muscle pressure *Pmus* varies dynamically. The processor 30 may take a maximum value of the respiratory muscle pressure *Pmus* as the characteristic quantity of the respiratory muscle pressure; or calculate an area *PTPmus* that is enclosed by the respiratory muscle pressure *Pmus* and a time axis, and take said area *PTPmus* as the characteristic quantity of the respiratory muscle pressure, wherein said area *PTPmus* corresponds to work done by the respiratory muscle pressure *Pmus,* and is capable of reflecting an intensity of a respiratory effort, for example, said area *PTPmus* can be obtaining by integrating the respiratory muscle pressure *Pmus* with respect to a time period from a start time point of an inspiration phase to an end time point of the inspiration phase.

The aforesaid respiratory mechanics equation is not originally proposed in this disclosure, and has been disclosed in a patent titled "*Medical Device and Method for Detecting Positive End-Expiratory Pressure"* with Application No. 202280063115.8. However, such patent adopts the respiratory mechanics equation and the system of equations to calculate the total positive end-expiratory pressure PEEPtot. In contrast, this disclosure aims to solve the respiratory muscle pressure *Pmus*, and further quantifies a respiratory effort of the patient by using the maximum value of the respiratory muscle pressure *Pmus*, or by using the area *PTPmus* that is enclosed by the respiratory muscle pressure *Pmus* and the time axis, so as to provide a basis for an intensity control of diaphragmatic pacing. It can be seen that the cited patent does not disclose a solution of solving the respiratory muscle pressure *Pmus* via the system of equations, nor a scheme of taking as a quantitative value for the respiratory effort of the patient, the maximum value of the respiratory muscle pressure *Pmus* or the area *PTPmus* that is enclosed by the respiratory muscle pressure *Pmus* and the time axis. The first calculation mode of the respiratory drive parameter can adopt inherent parameters of a ventilator for calculation without additional data collection, thereby presenting a favorable real-time performance, and improving an accuracy of subsequent closed-loop control for diaphragmatic pacing.

In the second implementation mode, the ventilation parameter includes an esophageal pressure *Pes* and chest wall compliance *Ccw*; and the corresponding respiratory drive parameter includes a characteristic quantity of the respiratory muscle pressure.

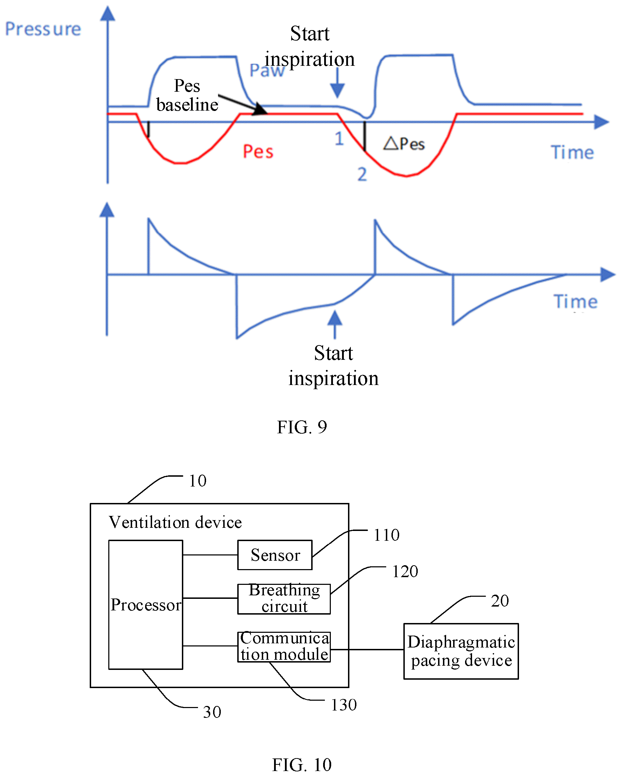

FIG. 8 is a schematic diagram of respiratory system modeling, in which: *Paw* refers to an airway pressure, *Flow* refers to a gas flow rate, *R* refers to respiratory system resistance, *P_{L}* refers to a transpulmonary pressure, *C_{L}* refers to lung compliance, *Ccw* refers to chest wall compliance, and *Pes* refers to an esophageal pressure, which is clinically adopted to approximate an intrathoracic pressure. *PEEPtot* refers to a total positive end-expiratory pressure, and *Pmus* refers to a respiratory muscle pressure generated by spontaneous respiration of patient. Waveform curves of the airway pressure *Paw*, the esophageal pressure *Pes* and the gas flow rate *Flow* are shown in FIG. 9.

Clinically, a change in the esophageal pressure *△Pes* or an area *PTPes* that is enclosed by the esophageal pressure *Pes* and a time axis can be used to evaluate a respiratory effort of the patient. Nevertheless, evaluating the respiratory effort through the change in the esophageal pressure *△Pes* or the area *PTPes* has two limitations. Firstly, a measurement of the esophageal pressure is complicated, time-consuming and labor-intensive. Secondly, evaluating the respiratory effort through the change in the esophageal pressure *△Pes* or the area *PTPes* has insufficient accuracy and is basically applied to qualitative assessment, failing to realize quantitative evaluation. However, in the second implementation mode provided by this disclosure, the processor 30 measures the chest wall compliance *Ccw* through the ventilation device 10. For example, the chest wall compliance *Ccw* can be acquired by performing one time of inspiratory hold and expiratory hold on the patient, and such measurement belongs to conventional technical means, which will not be elaborated herein. The processor 30 calculates the respiratory muscle pressure *Pmus* according to the esophageal pressure *Pes* and the chest wall compliance *Ccw*. Specifically, a ventilation volume *Volume* is acquired, and the respiratory muscle pressure *Pmus* is further calculated according to the ventilation volume *Volume,* the esophageal pressure *Pes* and the chest wall compliance *Ccw*. For example, the respiratory muscle pressure *Pmus* can be calculated through following formula: *Pmus=Pes-(Volume*/*Ccw)*. Furthermore, the processor 30 obtains the characteristic quantity of the respiratory muscle pressure based on the calculated respiratory muscle pressure *Pmus*. For instance, a maximum value of the calculated respiratory muscle pressure *Pmus* may be taken as the characteristic quantity of the respiratory muscle pressure, or an area *PTPmus,* which is enclosed by the respiratory muscle pressure *Pmus* and a time axis, can be calculated and taken as the characteristic quantity of the respiratory muscle pressure.

In the third implementation mode, the respiratory drive parameter includes an intensity of a phrenic electrical signal. The diaphragmatic electrical signal reflects a discharge amplitude of the diaphragm, and the electrical signal of the phrenic nerve reflects a movement amplitude of the diaphragm. The intensity or amplitude of the two signals directly indicates an amplitude of the respiratory drive and can quantitatively characterize a contraction strength of the diaphragm. Therefore, such signals are highly suitable as feedback parameters for diaphragmatic pacing, so as to realize a quantitative closed-loop control on diaphragmatic pacing, prevent poor training effect caused by an insufficient contraction amplitude of the diaphragm and diaphragm fatigue generated by excessive contraction amplitude of the diaphragm. Taking the diaphragmatic electrical signal as an example, a measurement catheter can be placed inside an esophagus of the patient, wherein the measurement catheter is arranged with a measurement electrode at its end, so as to measure the diaphragmatic electrical signal. The amplitude of the diaphragmatic electrical signal reflects the amplitude of the respiratory drive. Alternatively, an electrode pad that is attached to an abdomen of the patient can be configured to measure a surface diaphragmatic electromyography signal in a non-invasive manner, and the amplitude of the respiratory drive is quantified by calculating the amplitude of said signal. That is, the processor 30 can acquire the intensity (e.g., amplitude) of the phrenic electrical signal according to the collected phrenic electrical signal.

In Step 3, the processor 30 controls an intensity of the diaphragmatic pacing for the diaphragmatic pacing device 20 to perform the diaphragmatic pacing based on the respiratory drive parameter, so as to maintain the respiratory drive parameter within a preset range. Specifically, perform a closed-loop control on the intensity of the diaphragmatic pacing based on the respiratory drive parameter and the preset range, so as to maintain the respiratory drive parameter within a preset range. Performing a closed-loop control on the intensity of the diaphragmatic pacing specifically refers to performing a closed-loop control on an intensity of a stimulation signal that is outputted by the diaphragmatic pacing device 20.

The preset range corresponds to a normal range of the respiratory effort characterized by the respiratory drive parameter. When the respiratory effort of the patient is maintained within this preset range, the diaphragm of the patient can achieve an optimal training effect. The processor 30 performs a closed-loop feedback on the intensity of the stimulation signal according to the respiratory drive parameter, so as to stably maintain a contraction strength of the diaphragm within a clinically acceptable normal range, thus achieving an optimal training effect. The preset range includes a lower limit (value) and an upper limit (value), wherein a range between the lower limit and the upper limit is the preset range.

The processor 30 performing a closed-loop control on the intensity of the diaphragmatic pacing for the diaphragmatic pacing device 20 may include: the processor 30 decreases the intensity of the diaphragmatic pacing for the diaphragmatic pacing device 20, such as decreasing the intensity of the stimulation signal that is outputted by the diaphragmatic pacing device 20, when the respiratory drive parameter exceeds the upper limit of the preset range; the processor 30 increases the intensity of the diaphragmatic pacing for the diaphragmatic pacing device 20, such as increasing the intensity of the stimulation signal that is outputted by the diaphragmatic pacing device 20, when the respiratory drive parameter falls below the lower limit of the preset range. When the respiratory drive parameter is the characteristic quantity of the respiratory muscle pressure, a corresponding preset range may be 5~10 cmH₂O (centimeter of water). For example, the intensity of the diaphragmatic pacing for the diaphragmatic pacing device 20 is increased, if the respiratory muscle pressure *Pmus* is less than 5 cmH₂O, and the intensity of the diaphragmatic pacing for the diaphragmatic pacing device 20 is decreased, if the respiratory muscle pressure *Pmus* is greater than 10 cmH₂O. Apparently, the preset range corresponding to the characteristic quantity of the respiratory muscle pressure can also be other reasonable ranges and will not be restricted herein. When the respiratory drive parameter is an intensity of a diaphragmatic electrical signal, a corresponding preset range may be 5~30 µV (microvolt). For example, the diaphragmatic pacing device 20 increases the intensity of the stimulation signal for diaphragmatic pacing, if the intensity of the diaphragmatic electrical signal (such as a voltage amplitude) is less than 5 µV; while the diaphragmatic pacing device 20 decreases the intensity of the stimulation signal for diaphragmatic pacing, if the intensity of the diaphragmatic electrical signal is greater than 30 µV. Of course, the preset range corresponding to the intensity of the diaphragmatic electrical signal can also be other reasonable ranges and will not be restricted herein. When the respiratory drive parameter is an intensity of an electrical signal of a phrenic nerve, a corresponding preset range may be 100~200 µV. For example, the diaphragmatic pacing device 20 increases the intensity of the stimulation signal for diaphragmatic pacing, if the intensity of the electrical signal of the phrenic nerve (such as a voltage amplitude) is less than 100 µV; while the diaphragmatic pacing device 20 decreases the intensity of the stimulation signal for diaphragmatic pacing, if the intensity of the electrical signal of the phrenic nerve is greater than 200 µV. Of course, the preset range corresponding to the intensity of the electrical signal of the phrenic nerve can also be other reasonable ranges and will not be restricted herein.

To improve safety and prevent excessive stimulation, a maximum intensity may be preset to prevent the diaphragmatic pacing from being excessively high. When the respiratory drive parameter falls below a preset lower limit, the processor 30 increases an intensity of the diaphragmatic pacing for the diaphragmatic pacing device 20. Specifically, the processor 30 formulates a target intensity that is greater than a current intensity of the diaphragmatic pacing. Wherein the target intensity can be preset in advance, for example, by adding a fixed value to the current intensity. Afterwards, the processor 30 determines whether the target intensity exceeds a preset maximum intensity, that is, the processor 30 determines whether the target intensity exceeds a safety range, if yes, the processor 30 controls the diaphragmatic pacing device 20 to perform the diaphragmatic pacing according to the preset maximum intensity, such as to output a simulation signal that corresponds to the preset maximum intensity. That is, the intensity of the diaphragmatic pacing at most can be increased to the preset maximum intensity, so as to ensure the patient safe. If the processor 30 determines the target intensity does not exceed the preset maximum intensity, the processor 30 controls the diaphragmatic pacing device 20 to perform the diaphragmatic pacing according to the target intensity, such as to output a simulation signal that corresponds to the target intensity, so as to increase the intensity of the diaphragmatic pacing.

Performing the diaphragmatic pacing by the diaphragmatic pacing device 20 is a long-term continuous process. Although the aforesaid control method can train the diaphragm, while prevent diaphragm fatigue caused by excessive contraction. However, in order to avoid extreme case, this disclosure is further capable of directly determining whether the diaphragm is in a fatigue state, and adopting corresponding measure for such situation, which corresponding to form a dual protection mechanism for avoiding diaphragm fatigue. That is, the control method of the processor 30 integrates the procedures shown in FIG. 1 and FIG. 2. Specifically, in Step 5, the processor 30 acquires a respiratory drive parameter and/or a diaphragmatic electrical signal, which can be obtained through the aforementioned Step 1 and Step 2, and are not repeated herein. In Step 6 and Step 7, the processor 30 determines whether the diaphragm is in a fatigue state according to the respiratory drive parameter and/or the diaphragmatic electrical signal; and decreases the intensity of the diaphragmatic pacing, if the diaphragm is in the fatigue state; or continues the closed-loop control on the intensity of the diaphragmatic pacing (continuing performing procedures in FIG. 1), if the diaphragm is not in the fatigue state. Wherein there are various methods for determining whether the diaphragm is in a fatigue state, and two typical methods are described in detail below.

The first determination method is as follows. The processor 30 performs a spectral analysis on the diaphragmatic electrical signal, so as to obtain a low-frequency component *L* and a high-frequency component *H* of the diaphragmatic electrical signal. Low frequency and high frequency are relative concepts, which divide the signal into two parts according to a frequency characteristic. In this embodiment, the diaphragmatic electrical signal below 50 Hz is defined as a low-frequency component *L*, and the diaphragmatic electrical signal within 250-350 Hz is defined as a high-frequency component *H*. The processor 30 determines whether a ratio *H*/*L* of the high-frequency component to the low-frequency component is less than a preset first ratio, and then determines that the diaphragm is in the fatigue state, if said ratio is less than the preset first ratio, or determines that the diaphragm is in a normal state, if said ratio is equal to or greater than the preset first ratio. The preset first ratio can be set as a fixed value (e.g., 20%), which means fatigue determination for different patients is performed uniformly based on the first ratio. For instance, the processor 30 calculating the first ratio according to a preset range that corresponds to the intensity of the diaphragmatic electrical signal specifically includes: the processor 30 performs a spectral analysis on the diaphragmatic electrical signal corresponding to the preset range, so as to extract a low-frequency component and a high-frequency component of the diaphragmatic electrical signal; and takes a ratio of the high-frequency component to the low-frequency component as the first ratio. Alternatively, the first ratio may vary from patient to patient to enable individualized customization, allowing diaphragm fatigue assessment that better accommodates inter-individual differences. This embodiment is described based on this individualized scheme. When the diaphragmatic pacing device 20 starts to output a stimulation signal to the patient for diaphragmatic pacing (e.g., for the first time), the processor 30 correspondingly acquires the diaphragmatic electrical signal at this time point, and then performs a spectral analysis on said diaphragmatic electrical signal, so as to extract a low-frequency component and a high-frequency component of said diaphragmatic electrical signal; and takes a ratio of said high-frequency component to said low-frequency component as the first ratio. In such a way, the first ratio varies from patient to patient. As the diaphragmatic pacing continues, the ratio of the high-frequency component *H* to the low-frequency component *L*, namely *H*/*L*, decreasing compared with an initial ratio (the first ratio), indicates that the spectral has a significant shift, that is the high-frequency component decreases. Such a shift determines that the diaphragm is no longer in the fatigue state.

In a second determination mode, the processor 30 obtains a ratio of the respiratory drive parameter to the intensity of the diaphragmatic electrical signal, for example, the ratio of the characteristic quantity of the respiratory muscle pressure to the intensity of the diaphragmatic electrical signal, i.e., a current ratio. The processor 30 further determines whether the diaphragm is in a fatigue state according to an amplitude of change for said ratio. The principle is to determine whether a trend of change for the respiratory effort (the characteristic quantity of the respiratory muscle pressure) is consistent with that of the diaphragmatic electrical signal. A consistent trend indicates the diaphragm is not in the fatigue state, while an inconsistent trend suggests the diaphragm is in the fatigue state. Essentially, the fatigue state is quantitatively evaluated by the ratio of the respiratory muscle pressure *Pmus* to the amplitude of the diaphragmatic electrical signal. In this embodiment, the amplitude of change mainly refers to a decreased amplitude of the ratio. Specifically, the processor 30 may compare a current ratio with an initial ratio to obtain the amplitude of change for the ratio. The initial ratio is calculated according to the respiratory drive parameter and the intensity of the diaphragmatic electrical signal at a time point when starting to output a stimulation signal to the patient. That is, when the diaphragmatic pacing device 20 starts to output a stimulation signal to the patient for diaphragmatic pacing (e.g., for the first time), the processor 30 acquires a corresponding ventilation parameter and/or a diaphragmatic electrical signal, and then obtains a respiratory drive parameter according to said ventilation parameter or said diaphragmatic electrical signal that is acquired when the diaphragmatic pacing device 20 starts to output the stimulation signal for diaphragmatic pacing (the detailed process is described in the foregoing steps and will not be repeated herein), and takes a ratio of said respiratory drive parameter to the intensity of said diaphragmatic electrical signal, as the initial ratio, wherein said parameter and signal are acquired, when the diaphragmatic pacing device 20 starts to output a stimulation signal for diaphragmatic pacing. In this way, the initial ratio varies from patient to patient, realizing individualized determination of diaphragm fatigue. Alternatively, the processor 30 may compare the current ratio of the respiratory drive parameter to the diaphragmatic electrical signal intensity with a preset reference ratio to obtain the amplitude of change for the ratio. The reference ratio is a fixed value that is configured according to a user requirement, for example, calculated according to clinical experience. This mode adopts a unified reference ratio for all patients.

After acquiring the amplitude of change for the ratio, the processor 30 determines whether the amplitude of change exceeds a preset threshold (configurable as required, for example, 20%). The diaphragm is determined to be in a fatigue state, if the amplitude of change exceeds the preset threshold; otherwise, the diaphragm is determined to be in a normal state.

In Step 7, after determining that the diaphragm is in the fatigue state, the processor 30 decreases the intensity of the diaphragmatic pacing. Specifically, the processor 30 may control the diaphragmatic pacing device 20 to stop outputting the stimulation signal, that is, to suspend the diaphragmatic pacing, or to gradually decrease the intensity of the diaphragmatic pacing. This embodiment takes the gradually decreasing mode as an example, wherein the amplitude of the stimulation signal is decreased by a fixed value, so as to gradually decrease the intensity of the diaphragmatic pacing.

The processor 30 may further outputs prompt information for indicating diaphragm fatigue, when the diaphragm is in the fatigue state. The prompt information may be presented to a user in a form of sound, light or electricity. For example, the prompt information is displayed on a display of the ventilation device 10 and/or the diaphragmatic pacing device 20, so as to remind medical staff of the diaphragm fatigue of the patient.

The processor 30 may also suspend the closed-loop control on the intensity of the diaphragmatic pacing and decrease the intensity of the diaphragmatic pacing, after determining that the diaphragm is in the fatigue state. That is, when the diaphragm is in the fatigue state, the respiratory drive parameter no longer needs to be strictly maintained within the preset range, such that the intensity of the diaphragmatic pacing can be rapidly decreased to relieve diaphragm fatigue. Meanwhile, the procedure shown in FIG. 2 is continuously performed, that is continuing acquiring the respiratory drive parameter and/or the diaphragmatic electrical signal, so as to continuing determining whether the diaphragm is in the fatigue state, and determining that the diaphragm is no longer in the fatigue state, if the diaphragm is not in the fatigue state. After the diaphragm is no longer in the fatigue state, the processor 30 resumes the closed-loop control on the intensity of the diaphragmatic pacing, that is, the processor 30 continues to implement the procedure shown in FIG. 1, so as to maintain the respiratory drive parameter within the normal range, after the diaphragm is no longer in the fatigue state,

The method for controlling diaphragmatic pacing shown in FIG. 1 and FIG. 2 may be implemented by a ventilation system (referring the above embodiments) or by a ventilation device. As shown in FIG. 10, the ventilation device includes a sensor 110, a breathing circuit 120, a communication module 130 and a processor 30.

The sensor 110 is configured to acquire a ventilation parameter related to mechanical ventilation or to acquire a phrenic electrical signal of a patient. The sensor 110 may include a pressure sensor, a flow rate sensor, as well as a measurement catheter, a central venous catheter, an electrode pad and other components. The specific collection processes of ventilation parameter and phrenic electrical signal through the above components have been described in detail in the foregoing embodiments and will not be elaborated herein.

The breathing circuit 120 is configured to deliver a gas supplied by a gas source to the patient for mechanical ventilation. A gas loop, a hardware structure and a working principle of mechanical ventilation belong to conventional technical means and will not be repeated herein.

The communication module 130 is configured to establish a communication connection between the ventilation device and a diaphragmatic pacing device 20. The diaphragmatic pacing device 20 is configured to output a stimulation signal to the patient, so as to perform diaphragmatic pacing on the patient under a stimulation of the stimulation signal, wherein the specific functions thereof are described in the foregoing contents.

While the breathing circuit 120 performs the mechanical ventilation on the patient and the diaphragmatic pacing device 20 performs the diaphragmatic pacing on the patient, the processor is configured to obtain, via the sensor 110, the ventilation parameter related to mechanical ventilation or the diaphragmatic electrical signal of the patient. Of course, the communication module 130 may be connected with external devices such as a monitoring device, so that the processor 30 can also acquire a phrenic electrical signal from an external monitoring device (referring the above embodiments). The acquisition process for the ventilation parameter related to mechanical ventilation or the diaphragmatic electrical signal of the patient is consistent with Step 1 in the foregoing embodiments, and is not repeated herein.

The processor 30 obtains a respiratory drive parameter that characterizes a degree of a respiratory effort of the patient according to the ventilation parameter or the diaphragmatic electrical signal, the specific process may refers to the implementation process of Step 2, and is not repeated herein.

The processor 30 controls an intensity of the diaphragmatic pacing for the diaphragmatic pacing device 20 to perform the diaphragmatic pacing based on the respiratory drive parameter, so as to maintain the respiratory drive parameter within a preset range, the specific process may refers to the implementation process of Step 3, and is not repeated herein.

Moreover, the processor 30 may determine whether the diaphragm is in a fatigue state according to the respiratory drive parameter and the diaphragmatic electrical signal, and decrease the intensity of the diaphragmatic pacing for the diaphragmatic pacing device 20 to perform the diaphragmatic pacing, if the diaphragm is in the fatigue state, the specific process may refer to the above embodiments. Of course, the processor 30 may determine whether the diaphragm is in a fatigue state according to the diaphragmatic electrical signal, and decrease the intensity of the diaphragmatic pacing for the diaphragmatic pacing device 20 to perform the diaphragmatic pacing, if the diaphragm is in the fatigue state, the specific process may refers to the above embodiments.

The processor 30 taking the lead in controlling the ventilation device to control the diaphragmatic pacing, is just similar as that taking the lead in controlling the ventilation system to control diaphragmatic pacing, the specific process may refers to the foregoing embodiments, and is not repeated herein.

This disclosure is set forth with reference to various exemplary embodiments. However, those skilled in the art shall recognize that changes and modifications may be made to the exemplary embodiments without departing from the scope of the present disclosure. For example, various operation steps and components for executing such steps may be implemented in different manners according to specific applications or in consideration of any number of cost functions associated with system operation (for instance, one or more steps may be omitted, modified or combined with other steps).

In addition, as understood by those skilled in the art, the principles of the present disclosure may be embodied in a computer program product stored on a computer-readable storage medium, where computer-readable program codes are pre-stored in the storage medium. Any tangible and non-transitory computer-readable storage medium may be adopted, including magnetic storage devices (hard disks, floppy disks, etc.), optical storage devices (CD-ROMs, DVDs, Blu-ray discs, etc.), flash memories and the like. These computer program instructions may be loaded onto a general-purpose computer, a special-purpose computer or other programmable data processing equipment to form a machine, such that the instructions executed on the computer or other programmable data processing devices create apparatus for realizing specified functions. These computer program instructions may also be stored in a computer-readable memory that can direct a computer or other programmable data processing equipment to operate in a specific mode, so that the instructions stored in the computer-readable memory form an article of manufacture including implementation means for realizing specified functions. The computer program instructions may also be loaded onto a computer or other programmable data processing equipment, so as to execute a series of operation steps on the computer or other programmable devices to generate a computer-implemented process, such that the executed instructions provide steps for implementing the specified functions.

Although the principles of the present disclosure have been illustrated in various embodiments, numerous modifications in structures, layouts, proportions, elements, materials and components, which are particularly adapted to specific environments and operational requirements, may be adopted without departing from the principles and scope of the present disclosure. The above modifications and other alterations and amendments shall fall within the scope of the present disclosure.

The foregoing detailed description has been described with reference to various embodiments. Nevertheless, those skilled in the art will appreciate that various amendments and changes may be made without departing from the scope of the present disclosure. Accordingly, the content of the present disclosure shall be regarded as illustrative rather than restrictive, and all such modifications shall be covered within the scope thereof. Likewise, the advantages, other merits and solutions to problems of each embodiment have been described above. However, the benefits, advantages, problem solutions, and any elements generating or optimizing the same shall not be construed as critical, essential or indispensable. The term "including" and any variations thereof used in this document denote non-exclusive inclusion, such that a process, method, article or apparatus including a list of elements includes not only those elements, but also other elements that are not explicitly listed or inherent thereto. In addition, the term "connection" and any variations thereof as used herein refer to physical connection, electrical connection, magnetic connection, optical connection, communication connection, functional connection and/or any other form of connection.

## Claims

1. A computer-readable storage medium, on which a computer program is stored, wherein the computer program is capable of being executed by a processor, so as to implement a method for controlling diaphragmatic pacing, **characterized in that**, comprising:
while performing mechanical ventilation and diaphragmatic pacing on a patient, acquiring a ventilation parameter related to the mechanical ventilation or acquiring a phrenic electrical signal of the patient; wherein the phrenic electrical signal comprises a diaphragmatic electrical signal or an electrical signal of a phrenic nerve;
obtaining a respiratory drive parameter that characterizes a degree of a respiratory effort of the patient according to the ventilation parameter or the phrenic electrical signal; and
controlling an intensity of the diaphragmatic pacing based on the respiratory drive parameter, so as to maintain the respiratory drive parameter within a preset range.

2. The computer-readable storage medium according to claim 1, **characterized in that**, controlling an intensity of the diaphragmatic pacing based on the respiratory drive parameter, comprises:
performing a closed-loop control on the intensity of the diaphragmatic pacing based on the respiratory drive parameter and the preset range.

3. The computer-readable storage medium according to claim 1, **characterized in that**, the ventilation parameter comprises an airway pressure and a gas flow rate, while performing the mechanical ventilation on the patient; the respiratory drive parameter comprises a characteristic quantity of a respiratory muscle pressure;
obtaining a respiratory drive parameter that characterizes a degree of a respiratory effort of the patient according to the ventilation parameter, comprises:
obtaining a ventilation volume according to the gas flow rate; and
obtaining the characteristic quantity of the respiratory muscle pressure, according to a preset respiratory mechanics equation, the airway pressure, the gas flow rate and the ventilation volume; wherein the respiratory mechanics equation is constructed at least based on the airway pressure, the respiratory muscle pressure, the gas flow rate, the ventilation volume and a total positive end-expiratory pressure.

4. The computer-readable storage medium according to claim 3, **characterized in that**, in the respiratory mechanics equation, the respiratory muscle pressure has a preset functional relationship with time;
obtaining the characteristic quantity of the respiratory muscle pressure, according to a preset respiratory mechanics equation, the airway pressure, the gas flow rate and the ventilation volume, comprises:
substituting multiple groups of the airway pressure, the gas flow rate and the ventilation volume at different time points respectively into the preset respiratory mechanics equation, so as to establish a system of equations that corresponds to the respiratory muscle pressure and the total positive end-expiratory pressure;
solving the system of equations to obtain the respiratory muscle pressure, and obtaining the characteristic quantity of the respiratory muscle pressure according to the respiratory muscle pressure.

5. The computer-readable storage medium according to claim 1, **characterized in that**, the ventilation parameter comprises an esophageal pressure and chest wall compliance; the respiratory drive parameter comprises a characteristic quantity of a respiratory muscle pressure;
obtaining a respiratory drive parameter that characterizes a degree of a respiratory effort of the patient according to the ventilation parameter, comprises:
calculating the respiratory muscle pressure according to the esophageal pressure and the chest wall compliance, and obtaining the characteristic quantity of the respiratory muscle pressure according to the respiratory muscle pressure.

6. The computer-readable storage medium according to claim 1, **characterized in that**, the respiratory drive parameter comprises an intensity of the phrenic electrical signal;
obtaining a respiratory drive parameter that characterizes a degree of a respiratory effort of the patient according to the phrenic electrical signal, comprises:
obtaining the intensity of the phrenic electrical signal according to the phrenic electrical signal.

7. The computer-readable storage medium according to claim 6, **characterized in that**, acquiring a phrenic electrical signal of the patient, comprises:
measuring the diaphragmatic electrical signal of the patient via a measurement catheter that is placed inside an esophagus of the patient, when the phrenic electrical signal comprises the diaphragmatic electrical signal;
measuring the diaphragmatic electrical signal of the patient via an electrode pad that is attached to an abdomen of the patient, when the phrenic electrical signal comprises the diaphragmatic electrical signal;
measuring the electrical signal of the phrenic nerve of the patient via a central venous catheter with an electrode that is arranged on an outer wall of the central venous catheter, when the phrenic electrical signal comprises the electrical signal of the phrenic nerve;
measuring the electrical signal of the phrenic nerve of the patient via an electrode pad that is arranged at a position of a body surface of the patient, which position corresponds to the phrenic nerve of the patient, when the phrenic electrical signal comprises the electrical signal of the phrenic nerve.

8. The computer-readable storage medium according to claim 1, **characterized in that**, the phrenic electrical signal comprises the diaphragmatic electrical signal;
the method further comprises:
determining whether the diaphragm of the patient is in a fatigue state according to the respiratory drive parameter and/or the diaphragmatic electrical signal, and decreasing the intensity of the diaphragmatic pacing, if the diaphragm is in the fatigue state.

9. The computer-readable storage medium according to claim 2, **characterized in that**, the phrenic electrical signal comprises the diaphragmatic electrical signal; the method further comprises:
determining whether the diaphragm of the patient is in a fatigue state according to the respiratory drive parameter and/or the diaphragmatic electrical signal; and suspending the closed-loop control on the intensity of the diaphragmatic pacing and decreasing the intensity of the diaphragmatic pacing, if the diaphragm is in the fatigue state;
resuming the closed-loop control on the intensity of the diaphragmatic pacing, after the diaphragm is no longer in the fatigue state.

10. The computer-readable storage medium according to claim 2, **characterized in that**, performing a closed-loop control on the intensity of the diaphragmatic pacing based on the respiratory drive parameter and the preset range, comprises:
decreasing the intensity of the diaphragmatic pacing, when the respiratory drive parameter exceeds an upper limit of the preset range;
increasing the intensity of the diaphragmatic pacing, when the respiratory drive parameter falls below a lower limit of the preset range;
preferably, increasing the intensity of the diaphragmatic pacing, when the respiratory drive parameter falls below a lower limit of the preset range, comprises:
determining a target intensity that is greater than a current intensity of the diaphragmatic pacing, when the respiratory drive parameter falls below the lower limit of the preset range;
determining whether the target intensity exceeds a preset maximum intensity;
performing the diaphragmatic pacing according to the preset maximum intensity, so as to increase the intensity of the diaphragmatic pacing, if the target intensity exceeds the preset maximum intensity; or
performing the diaphragmatic pacing according to the target intensity, so as to increase the intensity of the diaphragmatic pacing, if the target intensity does not exceed the preset maximum intensity.

11. A computer-readable storage medium, on which a computer program is stored, the computer program is capable of being executed by a processor, so as to implement a method for controlling diaphragmatic pacing, **characterized in that**, comprising:
while performing mechanical ventilation and diaphragmatic pacing on a patient, acquiring a respiratory drive parameter that characterizes a degree of a respiratory effort of the patient and/or acquiring a diaphragmatic electrical signal of the patient;
determining whether the diaphragm of the patient is in a fatigue state according to the respiratory drive parameter and/or the diaphragmatic electrical signal, and decreasing an intensity of the diaphragmatic pacing, if the diaphragm is in the fatigue state.

12. The computer-readable storage medium according to claim 8 or claim 11, **characterized in that**, determining whether the diaphragm of the patient is in a fatigue state according to the respiratory drive parameter and/or the diaphragmatic electrical signal, comprises:
performing a spectral analysis on the diaphragmatic electrical signal, so as to obtain a low-frequency component and a high-frequency component of the diaphragmatic electrical signal; and
determining whether a ratio of the high-frequency component to the low-frequency component is less than a preset first ratio, and determining that the diaphragm is in the fatigue state, if said ratio is less than the preset first ratio.

13. The computer-readable storage medium according to claim 8 or claim 11, **characterized in that**, determining whether the diaphragm of the patient is in a fatigue state according to the respiratory drive parameter and/or the diaphragmatic electrical signal, comprises:
calculating a ratio of the respiratory drive parameter to the intensity of the diaphragmatic electrical signal; and
determining whether the diaphragm is in the fatigue state according to an amplitude of change for said ratio;
preferably, determining whether the diaphragm is in the fatigue state according to an amplitude of change for said ratio, comprises:
calculating the amplitude of change for said ratio by comparing said ratio with an initial ratio; or calculating the amplitude of change for said ratio by comparing said ratio with a preset reference ratio; wherein the initial ratio is calculated according to the respiratory drive parameter and the intensity of the diaphragmatic electrical signal when starting to output a stimulation signal to the patient;
determining that the diaphragm is in the fatigue state, when the amplitude of change exceeds a preset amplitude threshold.

14. A ventilation system, **characterized in that**, comprising a ventilation device, a diaphragmatic pacing device and a processor;
the ventilation device is configured to perform mechanical ventilation on a patient;
the diaphragmatic pacing device is configured to output a stimulation signal to the patient, so as to perform diaphragmatic pacing on the patient under a stimulation of the stimulation signal;
while the ventilation device performs the mechanical ventilation on the patient and the diaphragmatic pacing device performs the diaphragmatic pacing on the patient, the processor is configured to:
acquire a ventilation parameter related to the mechanical ventilation or acquire a phrenic electrical signal of the patient; wherein the phrenic electrical signal comprises a diaphragmatic electrical signal or an electrical signal of a phrenic nerve;
obtain a respiratory drive parameter that characterizes a degree of a respiratory effort of the patient according to the ventilation parameter or the phrenic electrical signal; and
control an intensity of the diaphragmatic pacing for the diaphragmatic pacing device to perform the diaphragmatic pacing based on the respiratory drive parameter, so as to maintain the respiratory drive parameter within a preset range.

15. A ventilation device, **characterized in that**, comprising:
a sensor, which is configured to acquire a ventilation parameter related to mechanical ventilation or acquire a phrenic electrical signal of a patient; wherein the phrenic electrical signal comprises a diaphragmatic electrical signal or an electrical signal of a phrenic nerve;
a breathing circuit, which is configured to deliver a gas supplied by a gas source to the patient for performing the mechanical ventilation;
a communication module, which is configured to establish a communication connection between the ventilation device and a diaphragmatic pacing device; wherein the diaphragmatic pacing device is configured to output a stimulation signal to the patient, so as to perform diaphragmatic pacing on the patient under a stimulation of the stimulation signal; and
a processor;
while the breathing circuit performs the mechanical ventilation on the patient and the diaphragmatic pacing device performs the diaphragmatic pacing on the patient, the processor is configured to:
acquire, via the sensor, the ventilation parameter related to the mechanical ventilation or the phrenic electrical signal of the patient;
obtain a respiratory drive parameter that characterizes a degree of a respiratory effort of the patient according to the ventilation parameter or the phrenic electrical signal; and
control an intensity of the diaphragmatic pacing for the diaphragmatic pacing device to perform the diaphragmatic pacing based on the respiratory drive parameter, so as to maintain the respiratory drive parameter within a preset range.
